# FASCICULE DE BREVET EUROPEEN

(11) **EP 1 442 759 B1**
(45) Date de publication et mention de la délivrance du brevet: **16.09.2009**
(21) Numéro de dépôt: 03293280.8
(22) Date de dépôt: 22.12.2003
(51) Int. Cl.: A61M 1/02, A61M 5/14

(54) **Système à poches de prélèvement à boucle préformée**
Beutelsystem zur Probenentnahme mit vorgeformter Schlaufe
Multiple bag system for probe extraction having a preformed loop

(30) Priorité: 03.02.2003 FR 0301196
(43) Date de publication de la demande: 04.08.2004
(73) Titulaire: MACO PHARMA, 59420 Mouvaux (FR)
(72) Inventeur: Behague, Maurice, 59126 Linselles (FR); Goudaliez, Francis, 59155 Faches-Thumesnil (FR); Verpoort, Thierry, 59420 Mouvaux (FR)
(74) Mandataire: Geismar, Thierry

(56) Documents cités:
- EP-A- 1 262 202
- WO-A-01/85029
- US-A- 5 309 604
- US-A- 5 352 371

## Description

L'invention concerne un système à poches pour le prélèvement d'un fluide biologique, et une machine de prélèvement dans laquelle un tel système est destiné à être utilisé pour permettre le prélèvement en circuit clos d'un fluide biologique additionné d'une solution anticoagulante et/ou de conservation.

Elle s'applique typiquement au cas où le fluide biologique est du sang total qui doit être prélevé d'un donneur dans une poche de recueil. En effet, il est recommandé que le sang soit prélevé de façon stérile et soit additionné d'une solution anticoagulante et/ou de conservation lors du prélèvement de sorte à permettre son utilisation ultérieure dans les meilleures conditions de sécurité sanitaire.

On connaît déjà, notamment du document FR-2 808 693 et du document US-4- 526 515, un système à poches pour le prélèvement du sang, qui comprend des moyens de prélèvement du fluide, une poche contenant une solution anticoagulante et/ou de conservation du fluide prélevé, et une poche de recueil destinée à recevoir le fluide prélevé additionné de la solution anticoagulante et/ou de conservation.

Pour permettre un prélèvement stérile du sang, la poche de recueil est en communication fluidique avec les moyens de prélèvement par l'intermédiaire d'une première tubulure souple et avec la poche contenant la solution anticoagulante et/ou de conservation par l'intermédiaire d'une deuxième tubulure souple, de sorte à former un système en circuit clos.

Ce type de système est destiné à être utilisé dans une machine de prélèvement comprenant une pompe péristaltique apte à écraser partiellement respectivement la première et la deuxième tubulure de sorte à permettre l'alimentation simultanée et en proportion contrôlée de la poche de recueil avec le sang et avec la solution anticoagulante et/ou de conservation.

A cet effet, l'opérateur doit en particulier disposer une partie de la deuxième tubulure autour d'une tête de la pompe de sorte à permettre l'alimentation de la poche de recueil en solution anticoagulante et/ou de conservation par écrasement partielle d'une zone de ladite deuxième tubulure.

Dans la pratique, cette disposition s'avère difficile et, dans le cas où elle est mal réalisée, le flux de solution pompée ne correspond pas à celui voulu. Il en résulte donc un possible déficit ou excès de solution anticoagulante et/ou de conservation dans la poche de recueil, ce qui est préjudiciable puisque cette solution doit être présente en quantité fixe pour que le sang recueilli soit utilisable dans le domaine médical.

L'invention a notamment pour but de résoudre cet inconvénient, en proposant un système à poches pour le prélèvement d'un fluide biologique en circuit clos, ledit système comprenant, préalablement à sa disposition dans la machine, une boucle préformée qui est agencée pour permettre sa disposition autour d'au moins une partie d'une tête d'une pompe péristaltique.

A cet effet, et selon un premier aspect, l'invention concerne un système à poches pour le prélèvement d'un fluide biologique, notamment sanguin, selon la revendication 1 ou selon la revendication 6.

Ainsi, l'opérateur doit disposer la boucle préformée autour de la tête de la pompe péristaltique pour permettre l'alimentation de la poche de recueil en solution anticoagulante et/ou de conservation, ce qui facilite le placement de façon appropriée de la deuxième tubulure dans la pompe. Outre la simplification de la manipulation, il en résulte une diminution du risque de mauvais positionnement et donc du risque d'obtenir une mauvaise concentration de solution anticoagulante et/ou de conservation dans la poche de recueil.

Selon une réalisation, le connecteur est formé d'une jonction trois voies sur lesquelles sont branchées respectivement l'extrémité aval d'une partie amont de la première tubulure, l'extrémité amont d'une partie aval de la première tubulure et l'extrémité aval de la deuxième tubulure. Le dispositif d'association est formé d'une pièce qui est pourvue d'un moyen d'association du dispositif sur la deuxième tubulure et d'un moyen d'association de la première tubulure sur la pièce.

Selon la revendication 6, le connecteur et le dispositif d'association sont formés d'une seule pièce. La pièce est par exemple formée d'une jonction cinq voies sur lesquelles sont branchées respectivement l'extrémité aval d'une partie amont de la première tubulure, l'extrémité amont d'une partie aval de la première tubulure, l'extrémité aval d'une partie amont de la deuxième tubulure, l'extrémité amont d'une partie aval de la deuxième tubulure et l'extrémité aval de la partie aval de la deuxième tubulure.

Selon un deuxième aspect, l'invention concerne une machine de prélèvement dans laquelle un système à poches selon l'invention est destiné à être utilisé pour permettre le prélèvement en circuit clos d'un fluide biologique additionné d'une solution anticoagulante et/ou de conservation. La machine comprend une pompe péristaltique pourvue d'une tête d'écrasement mobile en rotation et un dispositif de placement de la boucle autour d'une partie de ladite tête de sorte à permettre l'alimentation de la poche de recueil en solution anticoagulante et/ou de conservation par écrasement partiel d'une zone de la partie de la deuxième tubulure formant la boucle.

La machine comprend ainsi un dispositif de placement spécifique à la boucle préformée du système à poches, ce qui contribue encore à améliorer la facilité de manipulation et la précision du placement de la deuxième tubulure dans la machine. En outre, en faisant varier la vitesse de rotation de la tête, on peut obtenir un flux de solution anticoagulante et/ou de conservation déterminé, dans des conditions de stérilité optimales.

Selon une réalisation, la machine comprend une seule tête d'écrasement, le fluide étant prélevé par écoulement naturel, c'est-à-dire essentiellement par pression veineuse et par gravité, et la solution anticoagulante et/ ou de conservation est pompée pour être additionnée de façon proportionnelle et continue au sang prélevé.

Selon une réalisation, le dispositif de placement comprend une gorge agencée pour loger la partie de la boucle qui n'est pas disposée autour de la tête, ladite gorge comprenant au moins un logement pour le connecteur et/ou le dispositif d'association. En particulier, la géométrie d'au moins un logement peut être agencée pour permettre le blocage en translation du connecteur et/ou du dispositif d'association dans la gorge.

Cette réalisation, permet de limiter le glissement de la boucle lors de son écrasement par la tête, ce qui permet d'obtenir un meilleur contrôle de l'écoulement à l'intérieur de la deuxième tubulure, notamment du fait de l'amélioration de la constance du flux.

En variante, la gorge est agencée pour permettre le placement de la boucle dans un sens unique, ce qui évite tout risque d'erreur dans l'orientation de la boucle autour de la tête, qui conduirait par exemple à une alimentation des moyens de prélèvement avec la solution anticoagulante et/ou de conservation.

D'autres objets et avantages de l'invention apparaîtront au cours de la description qui suit en référence aux dessins annexés.

La figure 1 représente de façon schématique un système à poches selon un premier mode de réalisation de l'invention.

La figure 2 représente de façon schématique un système à poches selon un autre mode de réalisation de l'invention, la boucle dudit système étant disposée dans un dispositif de placement d'une machine de prélèvement suivant un mode de réalisation.

La figure 3 représente une vue en perspective du dispositif d'association de la deuxième tubulure sur la première tubulure d'un système à poches selon la figure 1.

La figure 4 représente de façon schématique un système à poches selon un deuxième mode de réalisation de l'invention, dans lequel le connecteur et le dispositif d'association sont formés d'une seule pièce.

La figure 5 représente une vue schématique en perspective d'une machine de prélèvement dans laquelle un système à poches selon l'invention est destiné à être utilisé pour permettre le prélèvement en circuit clos d'un fluide biologique additionné d'une solution anticoagulante et/ou de conservation.

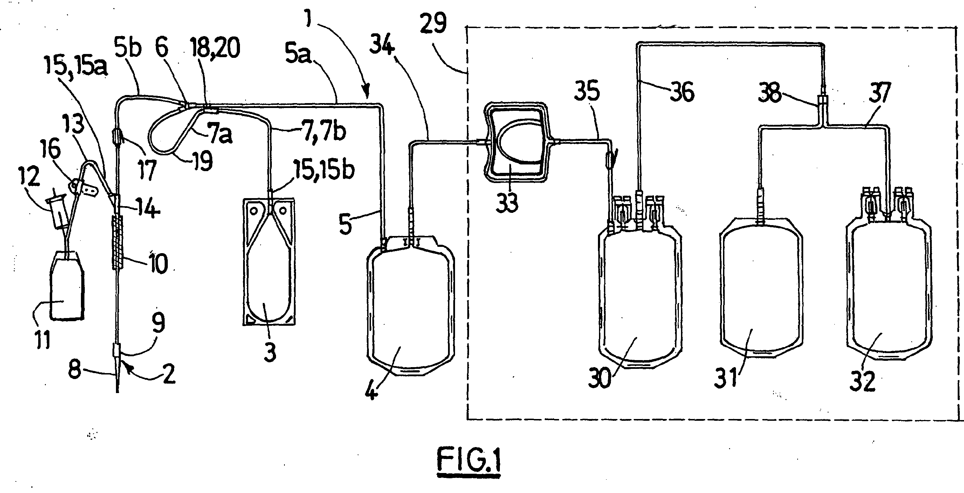

En relation avec la figure 1, un système à poches 1 comprend des moyens de prélèvement 2 d'un fluide tel que le sang d'un donneur, une poche 3 contenant une solution anticoagulante et/ou de conservation du fluide prélevé, tel qu'une solution de type ACD (acide citrate dextrose) ou CPD (citrate phosphate dextrose), et au moins une poche de recueil 4 destinée à recevoir le fluide prélevé additionné de la solution anticoagulante et/ou de conservation. La poche de recueil 4 est en communication fluidique avec les moyens de prélèvement par l'intermédiaire d'une première tubulure 5. La poche 3 contenant une solution anticoagulante et/ou de conservation est en communication fluidique avec ladite première tubulure au niveau d'un connecteur 6, par l'intermédiaire d'une deuxième tubulure 7. La première tubulure 5 et la deuxième tubulure 7 ont par exemple des sections identiques.

Tous les éléments d'un tel système à poches 1 sont pré-connectés de sorte à former un système unitaire fermé ou en circuit clos. Le système ainsi formé est donc prêt-à-l'emploi. En particulier, la poche de solution anticoagulante et/ou de conservation étant connectée de fabrication, on évite une étape de connexion préalablement au prélèvement, qui constitue une manipulation supplémentaire entraînant une perte de temps et un risque de rupture de la stérilité du système.

Les poches et les tubulures du système sont réalisées notamment en un matériau thermoplastique souple et stérilisable tel que le polychlorure de vinyle. Toutes les tubulures sont notamment souples, sécables et soudables.

Par exemple, le système à poches 1 est stérilisé et conditionné dans un emballage stérile.

Les moyens de prélèvement sont constitués notamment d'une aiguille 8 permettant l'accès à la veine du donneur et d'un capuchon 9 protégeant l'aiguille 8. En outre, un protecteur d'aiguille 10 peut être placé de façon coulissante sur la première tubulure 5.

La première tubulure comprend une partie aval 5a comprise entre le connecteur 6 et la poche de recueil 4 et une partie amont 5b comprise entre les moyens de prélèvement 2 et le connecteur 6.

Selon un mode de réalisation représenté figure 1, le connecteur 6 est formé d'une jonction trois voies en forme de Y. Sur les deux branches du Y sont branchées respectivement l'extrémité aval de la partie amont 5b de la première tubulure 5 et l'extrémité aval de la deuxième tubulure 7. Sur la tige du Y est branchée l'extrémité amont de la partie aval 5a de la première tubulure 5. La première et la deuxième tubulures 5 et 7 sont ainsi en communication fluidique.

Selon une réalisation, la partie aval 5a de la première tubulure 5 comprise entre le connecteur 6 et la poche de recueil 4 est de longueur suffisante pour obtenir un mélange homogène entre le fluide prélevé et la solution anticoagulante et/ou de conservation, lorsque le mélange atteint l'orifice d'entrée de la poche de recueil 4. La longueur de cette partie 5a de la première tubulure est notamment supérieure à 15 cm, par exemple de l'ordre de 25 cm.

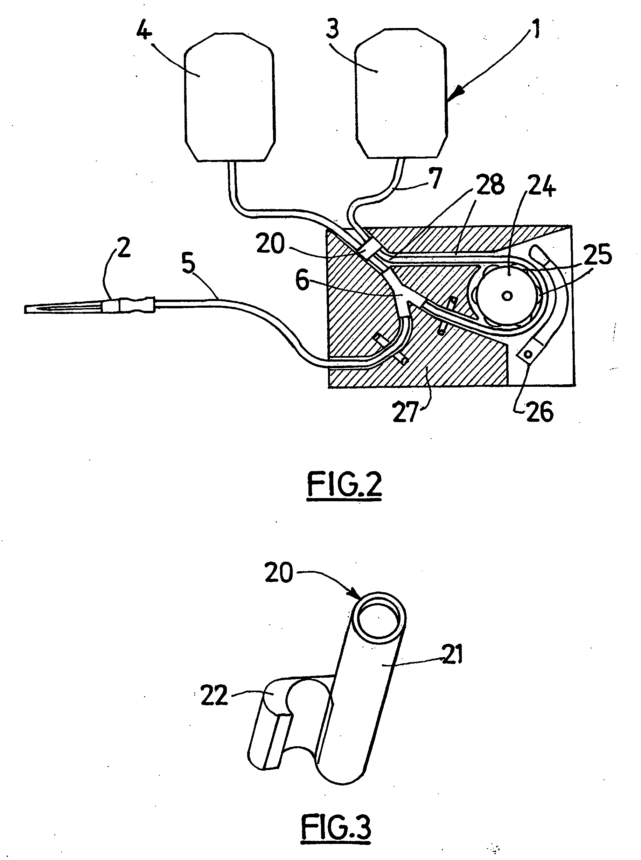

Selon un mode de réalisation et en relation avec les figures 1 et 3, le système à poches 1 comprend un dispositif d'association 18 formé d'une pièce 20 qui est pourvue d'un moyen d'association 21 du dispositif sur la deuxième tubulure 7 et d'un moyen d'association 22 de la première tubulure 5 sur la pièce 20.

La pièce 20 comprend un logement tubulaire formant moyen d'association 21 permettant la fixation non réversible de la pièce par insertion de la deuxième tubulure 7 dans ledit logement, et un logement latéral en forme de U formant moyen d'association 22 permettant la fixation réversible de la première tubulure 5 par clipsage de celle-ci dans ledit logement.

Selon une réalisation, la pièce 20 est associée à la première tubulure 5 par clipsage de la partie aval 5a dans le logement latéral.

Selon une variante, la première tubulure 5 est insérée dans le logement tubulaire formant moyen d'association 21 et la deuxième tubulure 7 est clipsée dans le logement latéral en forme de U formant moyen d'association 22.

La pièce peut être moulée dans un matériau plastique stérilisable, par exemple en polycarbonate.

La pièce 20 est agencée de manière à ce que la partie aval 7a de la deuxième tubulure 7, comprise entre le connecteur 6 et la pièce 20, forme une boucle 19 destinée à être disposée autour d'une tête d'écrasement d'une pompe péristaltique 24.

Selon une réalisation, la première tubulure 5 peut être clipsée dans le logement latéral en forme du U lors de la fabrication. En variante, la première tubulure peut être clipsée avant le prélèvement du liquide. Ceci permet d'adapter la taille de la boucle 19 en fonction des dimensions de la tête de pompe autour de laquelle la boucle est destinée à être disposée.

Dans le cas où les moyens d'association du dispositif 18 et/ou de la première tubulure 5 sont agencés pour permettre une fixation, l'assemblage des tubulures et de la pièce 20 servant de dispositif d'association 18 est réalisé, par exemple, par collage par solvant.

Selon un autre aspect représenté figure 1, le système à poches 1 comprend en outre un sous système d'échantillonnage sous la forme d'une poche d'échantillonnage 11 destinée à recevoir les premiers millilitres de sang prélevé et un dispositif d'échantillonnage latéral 12 associé à ladite poche d'échantillonnage 11 de sorte à permettre le prélèvement d'échantillons à l'aide de tubes sous vide.

Ladite poche d'échantillonnage est connectée par l'intermédiaire d'une troisième tubulure 13 et d'un connecteur 14, à la partie amont 5b de la première tubulure 5.

Les échantillons prélevés à partir de la poche d'échantillonnage 11 ne doivent pas contenir de solution anticoagulante et/ou de conservation afin de ne pas fausser les analyses effectuées ultérieurement sur ces échantillons.

Mais, on constate que lors de la stérilisation des système à poches, notamment par la vapeur d'eau, et sous l'effet de la pression créée à l'intérieur du système, les solutions contenues dans les poches desdits systèmes s'évacuent en dehors desdites poches.

Ainsi, la solution anticoagulante et/ou de conservation peut remonter de la poche 3 la contenant jusqu'à la poche d'échantillonnage 11.

Afin d'éviter cette remontée lors de la stérilisation du système, notamment par vapeur d'eau, il est avantageux de placer un obturateur de flux cassable 15 de type ouvre-circuit entre la poche 3 contenant la solution anticoagulante et/ou de conservation et ladite poche d'échantillonnage 11.

Par ailleurs, il est préférable que l'obturateur de flux 15 ne se trouve pas sur le passage d'écoulement du sang compris entre les moyens de prélèvement 2 et la poche de recueil 4 afin de prévenir tout risque d'hémolyse du sang prélevé au moment du prélèvement.

Un ouvre-circuit 15a est disposé sur la troisième tubulure 13 afin de bloquer l'accès à la poche d'échantillonnage 11 et sans gêner le flux du fluide prélevé lors du prélèvement.

Dans un aspect particulier, un ouvre-circuit 15b est disposé sur la partie amont 7b de la deuxième tubulure 7, notamment à proximité de son extrémité amont au niveau de l'orifice de sortie de la poche 3 contenant la solution anticoagulante et/ou de conservation. Ainsi, aucune trace de solution anticoagulante et/ou de conservation ne se trouve sur le passage d'écoulement compris entre les moyens de prélèvement 2 et la poche d'échantillonnage 11. Les échantillons prélevés sont alors exempts de solution anticoagulante et/ou de conservation et le flux du fluide prélevé n'est pas perturbé.

De même, avant utilisation du système à poches, aucune trace de solution anticoagulante et/ou de conservation ne remonte sur le chemin d'écoulement définit entre la poche de recueil 3 et les moyens de prélèvement 2, assurant l'exactitude du ratio entre la quantité de solution anticoagulante et/ou de conservation et la quantité de fluide prélevé.

En outre, des clamps 16, 17 peuvent être placés sur la première tubulure 5, précisément dans la partie amont 5b de la première tubulure, en amont du premier connecteur 6 et en aval du deuxième connecteur 14 et/ou sur la troisième tubulure 13, de sorte à contrôler le passage du fluide dans lesdites tubulures 5,13.

Selon un mode particulier de l'invention, un premier capteur optique peut être placé sur la première tubulure 5 entre les moyens de prélèvement 2 et le connecteur 6, de préférence entre les connecteurs 14 et 6. Ce capteur permet de détecter la présence de sang, afin de vérifier que le sang circule convenablement au sein de la tubulure 5. Il permet également de vérifier s'il n'y a pas d'air ou de solution anticoagulante et/ou de conservation remontant vers les moyens de prélèvement 2, donc vers le donneur. Ce capteur optique peut par exemple être remplacé et/ou complété par un capteur à ultrasons permettant de détecter de façon plus fine les inversions de flux. Un deuxième capteur optique peut être placé sur la deuxième tubulure 7. Ce capteur permet de détecter la présence de solution anticoagulante et/ou de conservation, afin de vérifier que la solution anticoagulante et/ou de conservation circule convenablement au sein de la tubulure 7. Ces capteurs sont connectés à une électronique de contrôle prévue sur la machine de prélèvement.

Selon un aspect particulier, le système à poches 1 est destiné à la séparation et à la filtration des composants sanguins en circuit clos.

Pour ce faire, le système à poches comprend en outre un sous-système 29 de traitement du fluide prélevé additionné d'une solution anticoagulante et/ou de conservation.

Le sous-système 29 comprend une ou plusieurs poche satellites 30, 31, 32 et au moins une unité de filtration 33 capable notamment de retenir les leucocytes en circuit clos. Le sous-système 29 est en communication fluidique avec la poche de recueil 4 par l'intermédiaire d'une tubulure.

Dans un exemple particulier représenté sur la figure 1, le système à poches 1 est fermé et destiné à la filtration du sang total. La poche de recueil 4 est reliée par l'intermédiaire d'une quatrième tubulure 34 à une unité de filtration 33, elle même reliée par l'intermédiaire d'une cinquième tubulure 35 à une poche de recueil du filtrat 30.

L'unité de filtration 33, par exemple du type décrit dans le document FR-2 677 883, permet l'élimination des leucocytes du sang total.

Une première poche satellite 31 contenant une solution de conservation des globules rouges de type SAGM, est reliée au moyen d'une sixième tubulure 36 à la poche de recueil du filtrat 30. Une seconde poche satellite 32 destinée à recevoir le plasma obtenu par centrifugation du sang total contenu dans la poche de recueil du filtrat 30 est en communication fluidique avec la poche de recueil du filtrat 30, au moyen d'une septième tubulure 37, par l'intermédiaire d'un troisième connecteur 38 placé sur la sixième tubulure 36.

Dans une variante non représentée, le système à poches est destiné à l'inactivation des pathogènes du sang ou des composant sanguins.

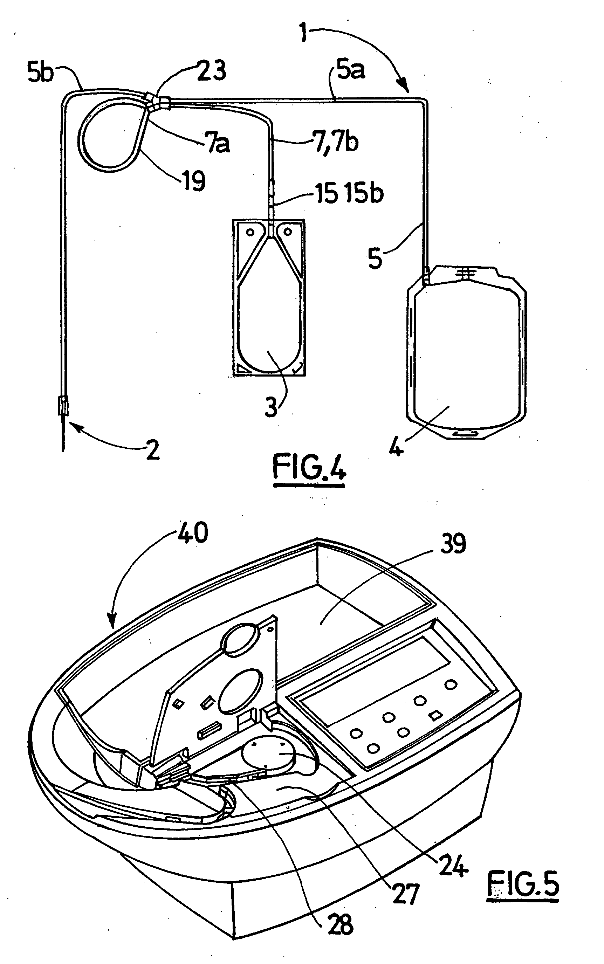

Selon un autre mode de réalisation représenté figure 4, le connecteur 6 et le dispositif d'association 18 sont réalisés d'une seule pièce 23 correspondant à l'association d'une jonction en Y et d'une jonction en I. Cette pièce 23 forme donc une jonction cinq voies à deux chemins d'écoulement distincts.

Dans ce mode de réalisation, la deuxième tubulure 7 est constituée de deux parties de tubulure, la partie aval 7a reliant les deux chemins d'écoulement et la partie amont 7b reliant la poche de solution anticoagulante et/ou de conservation 3 à la partie 7a par l'intermédiaire du deuxième chemin d'écoulement.

A cet effet, l'extrémité aval de la partie amont 5b, l'extrémité amont de la partie aval 5a et l'extrémité amont de la partie aval 7a sont respectivement branchées aux trois voies de la jonction en Y formant le premier circuit d'écoulement de la pièce 23. Par ailleurs, l'extrémité aval de la partie 7b et l'extrémité amont de la partie 7a sont respectivement branchées aux deux voies de la jonction en I formant le deuxième circuit d'écoulement de la pièce 23.

La boucle 19 formée par la partie aval 7a de la deuxième tubulure 7 est ainsi formée lors de la fabrication. Les dimensions de la boucle 19 sont alors définies au moment de la fabrication et restent inchangées par la suite.

En relation avec les figures 2 et 5, la boucle 19 est destinée à être placée autour d'au moins une partie d'une tête d'écrasement mobile en rotation d'une pompe péristaltique 24 d'une machine de prélèvement 40 de sorte à déplacer la solution anticoagulante et/ou de conservation dans ladite deuxième tubulure 7.

Par exemple, la machine de prélèvement 40 permet le prélèvement du sang par pression veineuse et par gravité assurant un mélange proportionnel entre le sang prélevé et la solution anticoagulante et/ou de conservation, et ce tout au long du prélèvement.

Une machine de prélèvement, représentée sur la figure 5, comprend :
- un dispositif de pesage 39 de type balance sur lequel on vient placer la poche de recueil 4 ou la poche de recueil 4 et la poche 3 de solution anticoagulante et/ou de conservation,
- une pompe péristaltique 24 comprenant une tête de pompe faisant circuler la solution la solution anticoagulante et/ou de conservation dans la deuxième tubulure,
- une électronique de pilotage (non représentée) qui contrôle, à partir des mesures de poids réalisées par le dispositif de pesage, la vitesse de rotation de la pompe péristaltique de façon obtenir la proportion volumique solution anticoagulante sur fluide prélevé désiré.

Lorsque le fluide prélevé est du sang d'un donneur, le ratio entre la quantité de solution anticoagulante et la quantité de sang prélevé est notamment de 1/7.

La partie aval 7a de la deuxième tubulure formant la boucle 19 peut présenter une dureté inférieure à celle de la première tubulure. En particulier, la deuxième tubulure 7 possède une dureté comprise entre 60 et 70 shore A, notamment 65 shore A. Les autres tubulures ont une dureté supérieure à 70 shore A, notamment 78.

Lorsque la pompe péristaltique 24 est en mouvement, les galets de la tête de pompe 24 viennent comprimer successivement la section de la deuxième tubulure 7 contre un dispositif courbe 26 de façon à assurer le déplacement de la solution anticoagulante et/ou de conservation.

Comme représenté sur la figure 2 ou 5, la tête de pompe est incluse dans une zone 27 d'aide au placement des tubulures. Cette zone 27 comprend une gorge 28 agencée pour loger la partie de la boucle 19 qui n'est pas disposée autour de la tête. Cette gorge 28 est, en outre, munie de logements permettant de loger le dispositif d'association 18 et le premier connecteur 6.

La géométrie des logements est agencée pour permettre le blocage en translation du connecteur et du dispositif d'association dans la gorge 28 de sorte que la deuxième tubulure 7 n'est pas entraînée par la rotation de la tête de pompe 24 en fonctionnement. En particulier, pour obtenir ce blocage, il peut être souhaitable d'avoir une association fixe entre la première tubulure 5 et le dispositif d'association 18.

L'immobilisation de cette deuxième tubulure 7 permet d'obtenir un écoulement homogène à l'intérieur de ladite deuxième tubulure 7 et un fonctionnement optimal de la pompe péristaltique 24.

La boucle 19 préformée combinée au premier connecteur 6 et au dispositif d'association 18 fait également office de détrompeur puisqu'elle oblige l'utilisateur à placer le système à poches 1 selon une orientation déterminée correcte, la gorge 28 étant agencée pour permettre le placement de la boucle dans un sens unique.

## Revendications

1. Système à poches (1) pour le prélèvement d'un fluide biologique, notamment sanguin, ledit système étant destiné à être utilisé dans une machine de prélèvement (40) comprenant une pompe péristaltique (24), ledit système comprenant, en circuit clos, des moyens de prélèvement (2) du fluide, une poche (3) contenant une solution anticoagulante et/ou de conservation du fluide prélevé, et une poche de recueil (4) destinée à recevoir le fluide prélevé additionné de la solution anticoagulante et/ou de conservation, dans lequel la poche de recueil (4) est en communication fluidique avec les moyens de prélèvement (2) par l'intermédiaire d'une première tubulure (5) souple pourvue d'un connecteur (6) et avec la poche (3) contenant la solution anticoagulante et/ou de conservation par l'intermédiaire d'une deuxième tubulure (7) souple branchée sur ledit connecteur, ledit système comprenant en outre un dispositif d'association (18) des première (5) et deuxième (7) tubulures, **caractérisé en ce que** ledit dispositif d'association est formé d'une pièce (20) comprenant un logement tubulaire formant moyen d'association non réversible de la pièce par insertion de la deuxième tubulure (7) dans ledit logement, et un logement latéral en forme de U formant moyen d'association réversible (22) de la première tubulure (5) par clipsage de celle-ci dans ledit logement, ledit dispositif étant agencé de manière à ce que la partie aval (7a) de la deuxième tubulure (7) entre le connecteur (6) et le dispositif d'association (18), forme une boucle (19), la conformation de ladite boucle étant agencée pour permettre sa disposition autour d'au moins une partie d'une tête de la pompe péristaltique (24).

2. Système selon la revendication 1, **caractérisé en ce que** le connecteur (6) est formé d'une jonction trois voies sur lesquelles sont branchées respectivement l'extrémité aval d'une partie amont (5b) de la première tubulure (5), l'extrémité amont d'une partie aval (5a) de la première tubulure (5) et l'extrémité aval de la deuxième tubulure (7).

3. Système selon la revendication 2, **caractérisé en ce que** la pièce (20) est obtenue par moulage d'un matériau plastique stérilisable.

4. Système selon la revendication 2 ou 3, **caractérisé en ce que** les moyens d'association du dispositif (18) et/ou de la première tubulure (5) sont agencés pour permettre une fixation.

5. Système selon la revendication 1, **caractérisé en ce que** la pièce (20) est associée à la première tubulure (5) par clipsage de la partie aval (5a) dans le logement latéral.

6. Système à poches (1) pour le prélèvement d'un fluide biologique, notamment sanguin, ledit système étant destiné à être utilisé dans une machine de prélèvement (40) comprenant une pompe péristaltique (24), ledit système comprenant, en circuit clos, des moyens de prélèvement (2) du fluide, une poche (3) contenant une solution anticoagulante et/ou de conservation du fluide prélevé, et une poche de recueil (4) destinée à recevoir le fluide prélevé additionné de la solution anticoagulante et/ou de conservation, dans lequel la poche de recueil (4) est en communication fluidique avec les moyens de prélèvement (2) par l'intermédiaire d'une première tubulure (5) souple pourvue d'un connecteur (6) et avec la poche (3) contenant la solution anticoagulante et/ou de conservation par l'intermédiaire d'une deuxième tubulure (7) souple branchée sur ledit connecteur, ledit système comprenant en outre un dispositif d'association (18) des première (5) et deuxième (7) tubulures, **caractérisé en ce que** le connecteur (6) et le dispositif d'association (18) sont formés d'une seule pièce (23), ledit dispositif étant agencé de manière à ce que la partie aval (7a) de la deuxième tubulure (7) entre le connecteur (6) et le dispositif d'association (18), forme une boucle (19), la conformation de ladite boucle étant agencée pour permettre sa disposition autour d'au moins une partie d'une tête de la pompe péristaltique (24).

7. Système selon la revendication 6, **caractérisé en ce que** la pièce (23) est formée d'une jonction cinq voies à deux chemins d'écoulement distincts.

8. Système selon l'une quelconque des revendications 1 à 7, **caractérisé en ce que** la deuxième tubulure (7) est pourvue-d'un ouvre circuit (15b) qui est disposé à proximité de son extrémité amont.

9. Système selon l'une quelconque des revendications 1 à 8, **caractérisé en ce que** la longueur de la première tubulure (5) mesurée entre le connecteur (6) et l'orifice d'entrée de la poche de recueil (4) est supérieure à 15 cm, notamment de l'ordre de 25 cm.

10. Système selon l'une quelconque des revendications 1 à 9, **caractérisé en ce qu'**au moins la partie de la deuxième tubulure (7) formant la boucle (19) présente une dureté inférieure à celle de la première tubulure (5).

11. Système selon l'une quelconque des revendications 1 :à 10, **caractérisé en ce qu'**il comprend en outre un sous système (29) de traitement du fluide additionné de la solution anticoagulante et/ou de conservation, ledit sous système comprenant des poches satellites (30, 31, 32) et éventuellement un ou des filtres (33) en circuit clos, ledit sous système étant en communication fluidique avec la poche de recueil (4) par l'intermédiaire d'une tubulure (34).

12. Système selon l'une quelconque des revendications 1 à 11, **caractérisé en ce qu'**il comprend en outre un sous système d'échantillonnage (11) du fluide prélevé, ledit sous système étant connecté à la première tubulure (5) en amont du connecteur (6).

13. Machine de prélèvement (40) dans laquelle un système à poches (1) selon l'une quelconque des revendications 1 à 12 est destiné à être utilisé pour permettre le prélèvement en circuit clos d'un fluide biologique additionné d'une solution anticoagulante et/ou de conservation, ladite machine comprenant une pompe péristaltique (24) pourvue d'une tête d'écrasement mobile en rotation et un dispositif de placement de la boucle (19) autour d'une partie de ladite tête, le placement de la boucle (19) autour de la tête de pompe permettant l'alimentation de la poche de recueil (4) en solution anticoagulante et/ou de conservation par écrasement partiel d'une zone de la partie de la deuxième tubulure (7) formant la boucle (19), **caractérisée en ce que** ledit dispositif de placement comprend une gorge (28) agencée pour loger la partie de la boucle (19) qui n'est pas disposée autour de la tête, ladite gorge comprenant un logement pour le connecteur et/ou le dispositif d'association (18).

14. Machine selon la revendication 13, **caractérisée en ce qu'**elle comprend une seule tête d'écrasement, le fluide étant prélevé par écoulement naturel.

15. Machine selon la revendication 14, **caractérisée en ce que** la géométrie d'au moins un logement est agencée pour permettre le blocage en translation du connecteur et/ou du dispositif d'association dans la gorge (28).

16. Machine selon la revendication 14 ou 15, **caractérisée en ce que** la gorge (28) est agencée pour permettre le placement de la boucle dans un sens unique.

## Claims

1. A system of bags (1) for sampling a biological fluid, more particularly blood, such system being intended to be used in a sampling machine (40) including a peristaltic pump (24), said system including, in a closed circuit, means for sampling (2) the fluid, a bag (3) containing a sampled fluid anticoagulant and/or preservation solution, and a collection bag (4) intended to receive the sampled fluid to which the anticoagulant and/or preservation solution has been added, wherein the collection bag (4) is in fluidic communication with the sampling means (2) through a first flexible tubing (5) provided with a connector (6) and with the bag (3) containing the anticoagulant and/or preservation solution through a second flexible tubing (7) connected to said connector, such system further comprising a device for associating (18) first (5) and second tubings (7), **characterised in that** said association device is formed in one piece (20) comprising a tubular recess forming a non-reversible association means for the part by inserting the second tubing (7) in said recess, and a U-shaped side recess forming a reversible association means (22) for the first tubing (5) by clamping the latter in said recess, said device being so arranged that the downstream part (7a) of the second tubing (7) between the connector (6) and the association device (18) forms a loop (19), with the configuration of said loop being so arranged as to allow the positioning thereof around at least one part of the head of the peristaltic pump (24).

2. A system according to claim 1, **characterised in that** the connector (6) is composed of a three-way junction whereon the downstream end of an upstream part (5b) of the first tubing (5), the upstream end of the downstream part (5a) of the first tubing (5) and the downstream end of the second tubing (7) are respectively connected.

3. A system according to claim 2, **characterised in that** the part (20) is obtained by moulding a sterilisable plastic material.

4. A system according to claim 2 or 3, **characterised in that** the means for associating the device (18) and/or the first tubing (5) are so arranged as to enable the fastening thereof.

5. A system according to claim 1, **characterised in that** the part (20) is associated to the first tubing (5) by clamping the downstream part (5a) in the side recess.

6. A system of bags (1) for the sampling of a biological fluid, more particularly blood, said system being intended to be used in a sampling machine (40) including a peristaltic pump (24), said system including, in a closed circuit, means for sampling (2) the fluid, a bag (3) containing a sampled fluid anticoagulant and/or preservation solution, and a collection bag (4) intended to receive the sample fluid to which the anticoagulant and/or preservation solution has been added, wherein the collection bag (4) is in fluidic communication with the sampling means (2) through a first flexible tubing (5) provided with a connector (6) and with the bag (3) containing the anticoagulant and/or preservation solution through a second flexible tubing (7) connected to said connector, said system further including a device for associating (18) the first (5) and second tubings (7), **characterised in that** the connector (6) and the association device (18) are formed in one piece (23), said device being so arranged that the downstream part (7a) of the second tubing (7) between the connector (6) and the association device (18) forms a loop (19), the configuration of said loop being so arranged as to allow the positioning thereof around at least one part of the head of the peristaltic pump (24).

7. A system according to claim 6, **characterised in that** the part (23) is formed of a five-way junction with two distinct outflow paths.

8. A system according to any one of claims 1 to 7, **characterised in that** the second tubing (7) is provided with a circuit opener (15b) which is positioned close to the upstream end thereof.

9. A system according to any one of claims 1 to 8, **characterised in that** the length of the first tubing (5) measured between the connector (6) and the inlet to the connection bag (4) is greater than 15cm, more particularly of the order of 25cm.

10. A system according to any one of claims 1 to 9, **characterised in that** at least the part of the second tubing (7) forming the loop (19) has a hardness which is less than that of the first tubing (5).

11. A system according to any one of claims 1 to 10, **characterised in that** it further includes a sub-system (29) for the processing of a fluid to which the anticoagulant and/or preservation solution has been added, said sub-system comprising satellite bags (30, 31, 32) and possibly one or several closed circuit filters (33), said sub-system being in fluidic communication with the collection bag (4) through a tubing (34).

12. A system according to any one of claims 1 to 11, **characterised in that** it further includes a sub-system for sampling (11) the sampled fluid, said sub-system being connected to the first tubing (5) upstream of the connector (6) .

13. A sampling machine (40) wherein a system of bags (1) according to any one of claims 1 to 12 is intended to be used for allowing the sampling in closed circuit of a biological fluid to which an anticoagulant and/or preservation solution has been added, said machine including a peristaltic pump (24) provided with a crushing head movable in rotation and a system for positioning the loop (19) around a part of said head, the positioning of the loop (19) around the pump head making it possible to supply the collection bag (4) with an anticoagulant and/or preservation solution by partially crushing an area of the part of the second tubing (7) forming the loop (19), **characterised in that** said positioning system includes a groove (28) arranged for receiving the part of the loop (19) which is not positioned around the head, said groove including a recess for the connector and/or the association device (18).

14. A machine according to claim 13, **characterised in that** it includes only one crushing head, with the fluid being sampled through a natural flow.

15. A machine according to claim 14, **characterised in that** the geometry of at least one recess is so arranged as to allow the locking in translation of the connector and/or the association device in the groove (28).

16. A machine according to claim 14 or 15, **characterised in that** the groove (28) is so arranged as to allow the positioning of the loop in only one direction.

## Patentansprüche

1. Beutelsystem (1) für die Entnahme einer biologischen Flüssigkeit, insbesondere Blut, wobei das genannte System dazu bestimmt ist, in einer Entnahmemaschine (40) verwendet zu werden, die eine peristaltische Pumpe (24) umfasst, wobei das genannte System im geschlossenen Kreislauf Entnahmemittel (2) der Flüssigkeit, einen eine antikoagulierende und / oder Konservierungslösung der entnommenen Flüssigkeit enthaltenden Beutel (3) und einen Aufnahmebeutel (4) umfasst, der dazu bestimmt ist, die entnommene, der antikoagulierenden und / oder Konservierungslösung zugesetzte Flüssigkeit aufzunehmen, in dem der Aufnahmebeutel (4) sich mit den Entnahmemitteln (2) über eine erste elastische Röhre (5), die mit einem Anschlussteil (6) versehen ist, und dem Beutel (3), der die antikoagulierende und / oder Konservierungslösung enthält, über eine zweite, an dem genannten Anschlussteil angeschlossene elastische Röhre (7) in flüssiger Verbindung befindet, wobei das genannte System darüber hinaus eine Verbindungsvorrichtung (18) der ersten (5) und zweiten (7) Röhren umfasst, **dadurch gekennzeichnet, dass** die genannte Verbindungsvorrichtung aus einem Stück (20) geformt ist, das eine röhrenförmige Aufnahme umfasst, die ein Mittel zur irreversiblen Verbindung des Stücks durch Einfügen der zweiten Röhren (7) in die genannte Aufnahme formt, und eine laterale Aufnahme in U-Form, die ein Mittel zur reversiblen Verbindung (22) der ersten Röhre (5) durch Verklippen derselben in der genannten Aufnahme formt, wobei die genannte Vorrichtung derart angeordnet ist, dass der untere Teil (7a) der zweiten Röhre (7) zwischen dem Anschlussteil (6) und der Verbindungsvorrichtung (18) eine Schleife (19) formt, wobei die Anpassung der genannten Schleife derart angeordnet ist, dass sie ihre Anordnung um wenigstens einen Teil eines Kopfes der peristaltischen Pumpe (24) erlaubt.

2. System gemäß Anspruch 1, **dadurch gekennzeichnet, dass** das Anschlussteil (6) aus einer Drei-Wege-Verbindung geformt ist, auf denen jeweils das untere Ende eines oberen Teils (5b) der ersten Röhre (5), das obere Ende eines unteren Teils (5a) der ersten Röhre (5) und das untere Ende der zweiten Röhre (7) angeschlossen sind.

3. System gemäß Anspruch 2, **dadurch gekennzeichnet, dass** das Stück (20) per Abformen eines sterilisierbaren Plastikteils erhalten wird.

4. System gemäß Anspruch 2 oder 3, **dadurch gekennzeichnet, dass** die Verbindungsmittel der Vorrichtung (18) und / oder der ersten Röhre (5) angeordnet sind, um eine Befestigung zu erlauben.

5. System gemäß Anspruch 1, **dadurch gekennzeichnet, dass** das Stück (20) mit der ersten Röhre (5) durch Verklippen des unteren Teils (5a) in der lateralen Aufnahme verbunden ist.

6. Beutelsystem (1) für die Entnahme einer biologischen Flüssigkeit, insbesondere Blut, wobei das genannte System dazu bestimmt ist, in einer Entnahmemaschine (40) verwendet zu werden, die eine peristaltische Pumpe (24) umfasst, wobei das genannte System im geschlossenen Kreislauf Entnahmemittel (2) der Flüssigkeit, einen eine antikoagulierende und / oder Konservierungslösung der entnommenen Flüssigkeit enthaltenden Beutel (3) und einen Aufnahmebeutel (4) umfasst, der dazu bestimmt ist, die entnommene, der antikoagulierenden und / oder Konservierungslösung zugesetzte Flüssigkeit aufzunehmen, in dem der Aufnahmebeutel (4) sich mit den Entnahmemitteln (2) über eine erste elastische Röhre (5), die mit einem Anschlussteil (6) versehen ist, und dem Beutel (3), der die antikoagulierende und / oder Konservierungslösung enthält, über eine zweite, an dem genannten Anschlussteil angeschlossene elastische Röhre (7) in flüssiger Verbindung befindet, wobei das genannte System darüber hinaus eine Verbindungsvorrichtung (18) der ersten (5) und zweiten (7) Röhren umfasst, **dadurch gekennzeichnet, dass** das Anschlussteil (6) und die Verbindungsvorrichtung (18) aus einem einzigen Stück (23) geformt sind, wobei die genannte Vorrichtung derart angeordnet ist, dass der untere Teil (7a) der zweiten Röhre (7) zwischen dem Anschlussteil (6) und der Verbindungsvorrichtung (18) eine Schleife (19) formt, wobei die Anpassung der genannten Schleife derart angeordnet ist, dass sie ihre Anordnung um wenigsten einen Teil eines Kopfes der peristaltischen Pumpe (24) erlaubt.

7. System gemäß Anspruch 6, **dadurch gekennzeichnet, dass** das Stück (23) aus einer Fünf-Wege-Verbindung mit zwei unterschiedlichen Ablaufwegen geformt ist.

8. System gemäß Anspruch 1 bis 7, **dadurch gekennzeichnet, dass** die zweite Röhre (7) mit einer Kreislauföffnung (15b) versehen ist, die in der Nähe ihres oberen Endes angeordnet ist.

9. System gemäß Anspruch 1 bis 8, **dadurch gekennzeichnet, dass** die Länge der ersten Röhre (5), die zwischen dem Anschlussteil (6) und der Eingangsöffnung des Aufnahmebeutels (4) gemessen wird, größer ist als 15 cm, insbesondere in der Größenordnung von 25 cm.

10. System gemäß Anspruch 1 bis 9, **dadurch gekennzeichnet, dass** wenigstens der Teil der zweiten Röhre (7), der die Schleife (19) bildet, eine geringere Härte als die der ersten Röhre (5) aufweist.

11. System gemäß Anspruch 1 bis 10, **dadurch gekennzeichnet, dass** es darüber hinaus ein Teilsystem (29) zur Behandlung der der antikoagulierenden und / oder Konservierungslösung zugesetzten Flüssigkeit umfasst, wobei das genannte Teilsystem Satellitenbeutel (30, 31, 32) und eventuell einen oder mehrere Filter (33) im geschlossenen Kreislauf umfasst, wobei das genannte Teilsystem sich über eine Röhre (34) in flüssiger Verbindung mit dem Aufnahmebeutel (4) befindet.

12. System gemäß Anspruch 1 bis 11, **dadurch gekennzeichnet, dass** es darüber hinaus ein Probenteilsystem (11) der entnommenen Flüssigkeit umfasst, wobei das genannte Teilsystem an die erste Röhre (5) oberhalb des Anschlussstücks (6) angeschlossen ist.

13. Entnahmemaschine (40), in der ein Beutelsystem (1) gemäß Anspruch 1 bis 12 dazu bestimmt ist, verwendet zu werden, um die Entnahme im geschlossenen Kreislauf einer biologischen Flüssigkeit zu erlauben, die einer antikoagulierenden und / oder Konservierungslösung zugesetzt wird, wobei die genannte Maschine eine peristaltische Pumpe (24) umfasst, die mit einem in Rotation mobilen Druckkopf und einer Platzierungsvorrichtung der Schleife (19) um einen Teil des genannten Kopfes versehen ist, wobei die Platzierung der Schleife (19) um den Kopf der Pumpe die Versorgung des Aufnahmebeutels (4) mit der antikoagulierenden und / oder Konservierungslösung durch teilweises Drücken eines Bereichs des Teils der zweiten Röhre (7), die die Schleife (19) formt, erlaubt, **dadurch gekennzeichnet, dass** die genannte Platzierungsvorrichtung eine Auskehlung (28) umfasst, die angeordnet ist, um den Teil der Schleife (19), der nicht um den Kopf angeordnet ist, unterzubringen, wobei die genannte Auskehlung eine Aufnahme für das Anschlussteil und / oder die Verbindungsvorrichtung (18) umfasst.

14. Maschine gemäß Anspruch 13, **dadurch gekennzeichnet, dass** sie einen einzigen Druckkopf umfasst, wobei die Flüssigkeit durch natürliches Ablaufen entnommen wird.

15. Maschine gemäß Anspruch 14, **dadurch gekennzeichnet, dass** die Geometrie wenigstens einer Aufnahme angeordnet ist, um das Blockieren in Translation des Anschlussteils und / oder der Verbindungsvorrichtung in der Auskehlung (28) zu erlauben.

16. Maschine gemäß Anspruch 14 oder 15, **dadurch gekennzeichnet, dass** die Auskehlung (28) angeordnet ist, um die Platzierung der Schleife in einer einzigen Richtung zu erlauben.
